# EUROPEAN PATENT APPLICATION

(11) **EP 4 488 740 A1**
(43) Date of publication of application: **08.01.2025**
(21) Application number: 23183424.3
(22) Date of filing: 04.07.2023
(51) Int. Cl.: G02B 21/00, A61B 34/20, A61B 90/20, A61B 90/25

(54) **MEDICAL SURGICAL MICROSCOPE WITH TARGET AUTOFOCUS AND METHOD FOR ADJUSTING THE FOCUS OF A MEDICAL SURGICAL MICROSCOPE**

(71) Applicant: B. Braun New Ventures GmbH, 79110 Freiburg im Breisgau (DE)
(72) Inventor: BEYL, Tim, 79115 Freiburg im Breisgau (DE); STAWIASKI, Jean, 79199 Kirchzarten (DE)
(74) Representative: Winter, Brandl - Partnerschaft mbB

(57) **Abstract**

The present disclosure relates to a medical surgical microscope (1) for a surgical procedure on a patient (P), comprising: a moveable microscope head (2) with an optical system (4) with a focus system (6) for focus adjustment to a focal point (8), the microscope (1) being adapted to provide an optical magnification of a target surgical area and to generate a digital microscope image, a movable robotic microscope arm (16) that is connected to a microscope base (18) to which the movable microscope head (2) is connected that is adapted to adjust a position and/ or an orientation of the microscope head (2), and a control unit (22) that is at least adapted to control the focus system (6) and the movable robotic microscope arm (16). The control unit (22) is adapted to: detect a first focal point (8) of the focus system (6) in a first position and orientation of the microscope head (2), wherein the first focal point (8) is located in a first focal plane (36) that is perpendicular to the optical axis (10) of the microscope head (2) in the first position and/or orientation, move the microscope head (2) into a second position and/or orientation, compute a second focal point (8') by determining a second focal plane (36') that crosses the first focal point (8) and is perpendicular to the optical axis (10') of the microscope head (2') in the second position and/or orientation in which the second focal point (8') is located, and to adjust the focus of the focusing system (6) to the second focal point (8'). Besides, the disclosure relates to a method, a computer readable storage medium and a computer program.

## Description

### Technical field

The present disclosure relates to a medical surgical microscope for a surgical procedure on a patient, comprising a movable microscope head with an optical system with an actuable or controllable focus system for focus adjustment to a focal point in particular in the direction of a first optical axis, and to generate a digital microscope image, preferably by means of a microscope sensing unit. The surgical microscope further comprises a movable robotic microscope arm that is connected to a microscope base to which the movable microscope head is connected, in particular articulated or mounted, that is adapted to adjust a position and/or an orientation of the microscope head and a control unit that is at least adapted to control the focus system and the movable robotic microscope arm. The optical system is preferably adjustable relative to the microscope base. In addition, the disclosure relates to a focusing method and a computer-readable storage medium and computer program according to the preamble of the independent claims of the disclosure.

### Background of the disclosure

Surgical microscopes are standard devices used in surgical procedures, particularly in neurosurgery, spinal surgery and microsurgery. These surgical microscopes usually comprise an optical system with a focus system. Usually multiple lenses in serial configuration with at least one lens being translational movable within the lens system are used for a focus operation and serve as the focus system. After the "last" lens of the optical system (averted to the front lens) a sensing unit, such as a CMOS sensor, is used to detect the final image of the microscope and digitally provide the image. An operator/ surgeon can have the microscopic image/ the microscopic image magnified for visual display of the (digitally) recorded microscope image via a display device such as an OP-monitor.

In case the surgical microscopes have a movable microscope head that is controlled by a surgeon or medical professional, the microscope head is moved either manually or actively by a robotic microscope arm and with every movement, the focus must always be adjusted as a distance and orientation to a target surgical area or target point of interest and thus, the focus that needs to be adjusted changes. This focusing step is usually performed using a current captured optical microscope image while performing contrast or phase optimisation to adjust the focus/ focal length in order to obtain a sharp image of the target surgical area. This type of focusing is an iterative process and actuators are usually used to perform the automatic focusing (autofocusing). The disadvantage of this type of focusing is that the focusing process usually takes 0.2 to 2 seconds and is accompanied by an unstable microscope image during focus adjustment and noise generated by the actuators.

This complex and lengthy focusing step leads to an interruption of the operation and, if repeated several times, to a loss of concentration of the surgeon. Another problem arises when the direct view of the surgical target area is temporarily blocked by either by instruments or by fluid. Among other things, this results in an unintentional activation of the autofocus. Although a single one-time, (initial) focus adjustment or focusing step is relatively short (a few seconds) and does not take much time, the surgical microscope is continuously in motion during surgical procedures, therefore, constantly recurring temporary loss of image sharpness/ focus as well as the time needed for constant focus adjustment steps are very annoying and can lead to a significant impairment of the surgeon. For this reason, surgeons often even deactivate the autofocus or adjust the focus manually themselves, which in turn requires the additional (manual) operation by the surgeon and can delay a procedure. Constant focus adjustment when using a surgical microscope is therefore currently still associated with many complications and with an impairment of patient safety.

### Brief description of the disclosure

Therefore, the tasks and objectives of the present disclosure are to avoid or at least to reduce the disadvantages of the prior art and, in particular, to provide a medical surgical microscope, a focusing method as well as a computer-readable storage medium and computer program, which enable better, simpler, safer and more reliable focusing during a surgical procedure. Another task is to further increase a focus adjustment speed and to shorten the time of the surgical procedure for a surgeon as well as providing an even more interference-free autofocus of a surgical microscope.

The object of the present disclosure are achieved according to the present invention with regard to a medical surgical microscope with the features according to claim 1 and according to the present invention regarding the focusing method according to the features claim 11 as well as with regard to a computer-readable storage medium according to the features of claim 13 and with regard to a computer program according to the features of claim 14 of the disclosure.

Specifically, according to the present disclosure a medical surgical microscope for a surgical procedure on a patient is provided, comprising a moveable microscope head with an optical system with a focus system for focus adjustment to a focal point, the microscope being adapted to provide an optical magnification of a target surgical area, in particular in the direction of a first optical axis, and to generate a digital microscope image, preferably by means of a microscope sensing unit, a movable robotic microscope arm that is connected to a microscope base! robotic base to which the movable microscope head is connected that is adapted to adjust a position and/ or an orientation of the microscope head, and a control unit that is at least adapted to control the focus system and the movable robotic microscope arm. According to the disclosure, the control unit is adapted to detect a first focal point of the focus system in a first position and orientation of the microscope head. The first focal point is located in a first focal plane that is perpendicular to the optical axis of the microscope head in the first position and/or orientation, to move the microscope head into a second position and/or orientation, to compute a second focal point by determining a second focal plane that intersects/ crosses the first focal point and is perpendicular to the optical axis of the microscope head in the second position and/or orientation in which the second focal point is located, and to adjust the focus of the focusing system to the second focal point.

In other words, when the medical surgical microscope is moved by an operator/ surgeon during a procedure, specifically its microscope head with the optical system and the focus system, a focal plane/ focus plane is utilized. The normal of the focal plane is equal to the optical axis of the optical system and the focal plane also crosses the focal point, in other words, the focal point is located on the focal plane. After a change of position and/ or orientation of the microscope head, and in order to compute the (new) second focal point / the focus target for the focus system, preferably for the focus motors of the focus system, the focal plane is rotated around the original focal point so that the normal of the rotated (second) focal plane is equal to the new rotated optical axis. In other words, the focal plane rotates with the microscope head. For every point in time, the first focal point is projected on the point where the rotated focal plane and the rotated optical axis cross. Preferably, the surgical (robotic) microscope comprises more than one camera as part of the optical system. The one or more cameras can in one example be stereo cameras. Furthermore, non mechanical focussing cameras may preferably also be used, e.g. lightfield cameras. In this case, no (focus) motor moves but the correct focal plane is shown. More than one focal point can be selected at once and the focal point may not be on the optical axis of the optical system.

This enables the focus system of the surgical microscope to continuously keep a target surgical area in focus by localizing the focal point of the focus system, while a movement of the microscope head is taking place, for a given surgical target area and to adjust the focus of the new position/ orientation to the target surgical area. By calculating the focal points based on focal planes, no additional (external) tracking system and additional information of an external camera are needed since the focus adjustment is calculated based on the (internal robotic) kinematics/ movements of the surgical microscope, in particular of the microscope head. As the focus adjustment is taking place automatically during the procedure, the complexity as well as the time of the surgical procedure can be decreased. Additionally, this also eases the surgical procedure for the surgeon.

The term "target (surgical) area" is used here to describe a part of an intervention area that is of interest and is to be targeted and focused on. In particular, the target area can be a single target point in space (with X, Y, Z coordinates) and can also be specified as such coordinates. However, the target area can also be a section of a two-dimensional plane, such as a small section of a tissue surface. In particular, a central point of this plane is chosen as the (average) focal point. Also, a target area may be specified as a small cube-shaped or spherical volume, with the microscope focusing approximately on a center point of this volume (as a focal point), thus ensuring an "average" focus.

The term "focal point" defines a point in space which lies is should be lying in a focal plane of the optical system and in particular on the optical microscope axis.

The term "tracking system" describes a technical system that enables spatial localization and can detect a position and/or orientation.

The term "position" means a geometric position in three-dimensional space, specified in particular by means of coordinates of a Cartesian coordinate system. In particular, the position can be indicated by the three coordinates X, Y and Z.

The term "orientation", in turn, specifies an orientation in space. One can also say that by the orientation is indicated a direction or rotation in the three-dimensional space. In particular, and an orientation can be specified by means of three angles.

The term "pose" includes both a position and an orientation. In particular, the position can be specified by means of six coordinates, three position coordinates X, Y and Z as well as three angle coordinates for the orientation.

Further advantageous developments and embodiments of the disclosure are subject-matter of the dependent claims.

In a different preferred aspect of the disclosure, the control unit of the medical surgical microscope is adapted to transform the position of the first focal point of a local coordinate system of the microscope head into a local coordinate system of the microscope base, in order to continuously calculate the position of the first focal point relative to the microscope base. Preferably, the control unit is adapted to calculate the transformation of the first focal point of the local coordinate system of the microscope head into the local coordinate system of the microscope base by calculating a first transformation from the local coordinate system of the microscope head to a local coordinate system of a microscope flange, and calculate a second transformation from the microscope flange to the microscope base, in order to enhance the calculation process. In other words, the transformation of the first focal point to a position of the microscope base is based on a (coordinate) transformation from an optical center of the camera that is part of the optical system of the microscope head to the first focal point and a transformation from said optical center to the microscope base to which the (robotic) microscope arm is connected. The transformation from the optical center to the microscope base / base of the robot is composed of a calibration (or from CAD information) transform from the camera of the optical system to the robot flange and from the robot flange to the microscope base which is known at any time since the microscope arm is being send to a position and/ or since the transformation is computed by the control unit using the forwards kinematics and the joint angles of the microscope arm. Therefore, the current focal point is known in the microscope base / the information of the focal point relative to the original focal point is available to the control unit.

In another preferred embodiment of the present disclosure, the control unit of the surgical microscope is adapted to calculate a focal length by calculating a distance between the optical system of the microscope head and the focal plane in which the current focal point is located. In other words, the distance between the focal plane with the current focal point and the, preferably one, camera of the optical system of the microscope head is calculated by the control unit (continuously) at any time. Whenever the microscope head including the optical system is moved along the optical axis/ focal axis the control unit computes the distance between the focal point and the center of the optics of the camera of the optical system. Using this information the focus system, preferably the focus motors, can be controlled such that at any time the optical system of the microscope is focused on the selected focal point.

In a further preferred embodiment of the disclosure, the focus system of the surgical microscope is adapted to adjust the focus by explicitly setting the focus to the focal length/ distance of the second focal point calculated by the control unit. In other words, the control unit adjusts the focus of the focus system using feedforward information. Thus, the focus system is controlled using position control which means that the control unit signals the focus system where the current focal point is located and where it should focus on, preferably by activating the focus motors, thereby driving its focus to that point.

In a further preferred aspect of the disclosure, the control unit of the surgical microscope is adapted to set, based on a calculated focal length of the second position and/ or orientation of the microscope head, a minimum focal length and/ or a maximum focal length as focus limiters, and the control unit is adapted to perform a contrast focusing within the focus limiters. In other words, by setting a minimum and/or a maximum focal length, the initially computed focal length is usable as an initial seed for an autofocusing process in order to increase the speed and/ or reliability of the autofocusing process since the search space for the focus system gets limited. A focus range is therefore set and the final automatic fine tuning is executable by using contrast focusing.

In a further preferred embodiment of the disclosure, a minimum and a maximum focal length is set, wherein the minimum focal length and the maximum focal length are equidistant to the focal length calculated by the control unit, or a difference between the maximum focal length and the focal length is smaller than a difference between the focal length and the minimum focal length, or a difference between the maximum focal length and the focal length is larger/ greater than a difference between the focal length and the minimum focal length. In other words, the range in which the initially calculated focal length is set, thus the area in which the focal plane is located, can vary according to preselected preferences. Either the computed minimal and maximal focal length are equally distant from the initially computed focal length or one is close/ further to the computed focal length than the respective minimum or maximum focal length.

In a further preferred embodiment of the disclosure, the focus system of the surgical microscope is adapted to adjust the focus based on a velocity information of the (robotic) microscope head calculated by the control unit, preferably by using the first position and orientation of the microscope head and by, in particular continuously, integrating a (translator) velocity vector as well as a rotation/ rotatory velocity.

In a further preferred embodiment of the disclosure, the control unit of the surgical microscope is adapted to calculate the current focal length of the moving microscope head in real time and to continuously adjust the focal length of the focus system accordingly, in order to hold the focus while moving the microscope head. In other words, the control unit continuously signals the focus system to adjust the focus based on velocity information of the movement of the microscope head. Therefore, the focus adjustment takes place on-the-fly and no additional configuration step is needed compared to an adjustment based on a position control.

In a further preferred embodiment of the disclosure, the control unit of the surgical microscope is adapted to control a focus motor of the focus system via a velocity control or via a current control in order to adjust the focus of the focusing system, preferably to the second focal point. Further, the focus motor of the focus system may be a motor that is adapted to be controlled via velocity control or current controlled, preferably in the form of a brushless motor.

Preferably, the movable robotic microscope arm has a plurality of microscope arm segments that are connected to each other via a joint (two adjacent robotic arm segments) and can be controlled by the control unit to take a robotic microscope arm configuration.

The present disclosure further relates to a focus method for adjusting the focus on a target surgical area, in particular for a medical surgical. This method according to the present disclosure comprises the steps: Detecting a first focal point of a focus system of an optical system in a first position and orientation of a movable microscope head with a control unit, determining a first focal plane that crosses the first focal point and is perpendicular to an optical axis of the microscope head in the first position and/or orientation, focusing of a focusing system on the first focal point, moving the microscope head from the first position and/ or orientation to a second position and/ or orientation, computing a second focal point by determining a second focal plane that crosses the first focal point and is perpendicular to an optical axis of the microscope head in the second position and/ or orientation in which the second focal point is located, and adjusting the focus of the focusing system to the second focal point. With these steps, the focus can effectively and fast be adjusted.

In a further preferred embodiment of the disclosure, the focusing method comprises the additional steps calculating a focal length by calculating a distance between the optical system of the microscope head and the focal plane in which the first or second focal point is located and adjusting the focus by explicitly setting the focus to the focal length of the first or second focal point.

Preferably, the concept may be combined with any focusing techniques such as a focusing procedure on the center of the image or a sharpness maximizing process of the image over the entire image. In each case a focal plane exists that crosses the optical axis, as the focal point.

Preferably, the focusing algorithm may be part of a controller so that at any time when the microscope head is being moved, the target surgical area is kept in focus. Furthermore, the concept may be combined with optical autofocusing techniques so that also the target/ patient can move simultaneously with the microscope head/ microscope. Alternatively to moving a focus motor of the focus system, the microscope head may be moved on a trajectory so that a distance between the focal plane and the optical center of the optical system is constant, therefore, no motoric adjustment of the focus is needed. In addition, the focusing method may be used in combination with optical autofocusing of image processing techniques, so that a coarse/ broader focus position is computed from the microscope position and a fine focus positioning is performed with the optical focus system.

The present disclosure also relates to a computer readable storage medium as well as to a computer program each comprising instructions which, when executed by a computer, cause the computer to perform the focusing method of the present disclosure.

### Brief description of the drawings

In the following, the disclosure will be described in greater detail by means of the accompanying drawings. It is showing:
- Fig. 1: a perspective, schematic view of a medical surgical microscope according to a preferred embodiment of the disclosure,
- Fig. 2: a perspective view of the medical surgical microscope according to the preferred embodiment of the disclosure of Fig. 1 with an indicated transformation of the position of the first focal point of a local coordinate system of a microscope head into a local coordinate system of a microscope base,
- Fig. 3: a perspective view of the medical surgical microscope of the preferred embodiment of Figs. 1 and 2 of the disclosure with a first and a second position of the microscope head as well as a respective first and a second focal plane, and
- Fig. 4: a flow chart of a method for adjusting the focus according to a preferred embodiment of the present disclosure.

The figures are only of a schematic nature and are intended solely for the purpose of understanding the disclosure. Identical elements are marked with the same reference signs. The features of the different embodiments can be interchanged.

### Detailed description of the preferred embodiments

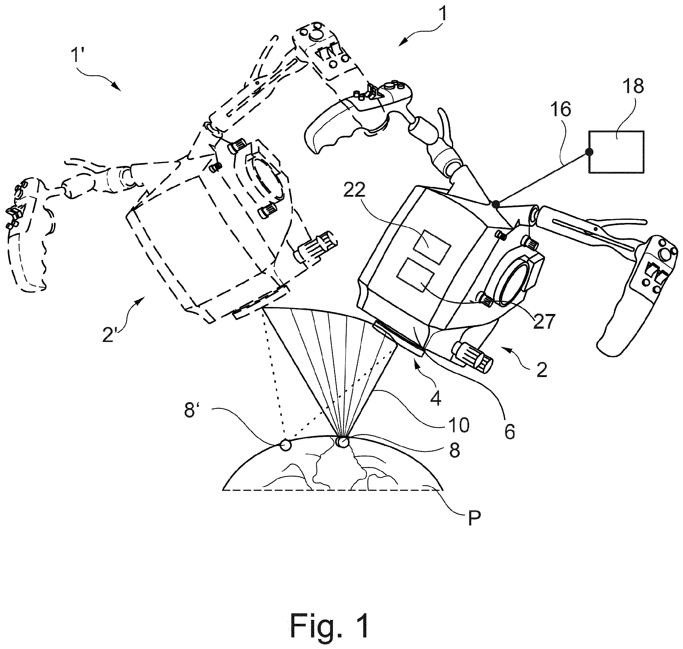

Fig. 1 shows a perspective, schematic view of a medical surgical microscope 1 (hereinafter referred to only as microscope) according to a preferred embodiment of the present disclosure, which is used in a surgical procedure on a patient P.

The microscope 1 has a movable microscope head 2 that is moved by an operator in order to microscope from different positions. The microscope head 2 has an optical system 4 with lenses, such as an objective and an eyepiece for multiplying the effects of the lenses to achieve high magnification. The optical system 4 further comprises a focus system 6, such as a mechanical system, to move the lenses relative to each other and to adjust a focus on a focal point 8. The optical system 4 provides an optical magnification of an area of the focal point 8, and a downstream CMOS sensor as a microscope imaging unit detects the optical magnification and creates a digital microscope image.

The movable microscope head 2 in Fig. 1 is connected to a microscope base 18 via a schematically shown (robotic) microscope arm 16 in the form of a strut with a ball bearing at each end of the strut, so that the microscope arm 16 is movable/movably mounted to the microscope base 18 and so that the microscope head 2 is also movably mounted relative to the microscope arm 16 and thus, relative to the microscope base 18. This configuration enables a change of its position (three coordinates) as well as a change of its orientation (orientation of the microscope head 2) and thus, the position of the microscope head 2 is freely changeable by the operator or automatically changeable. Since the optical system 4 with its front lens is fixed to the microscope head 2, a position of the optical system 4 can be adjusted by moving the microscope head 2.

A control unit 22 of the surgical microscope 1 detects a change in the position and in the orientation of the microscope head 2 and calculates the relative change of a position/orientation based on the (robot) kinematics of the microscope arm 16 and/or microscope head 2. Preferably, the control unit 22 calculates the position of the microscope head 2 relative to the position of a target focal point 8, preferably on an optical axis 10 of the optical system 4. Based on this information of the relative arrangement, the control unit 22 can control the focusing system 6 in such a way that the focusing system 6 focuses precisely the focal point 8 or new focal point 8'.

Specifically, the control unit 22 determines based on the position and orientation of the microscope head 2 a vector between the optical system 4 and the target surgical area or the focal point 8, 8'. The length of this vector corresponds to the focal length or focus to be adjusted by the focus system 6. Thereby, the focus is automatically set to the target surgical area.

Through this configuration of the microscope 1, the focus is directly determined and adjusted by the focus system 6. There is no need for an iterative readjustment process and the automatic focusing is instantaneous, so to speak. A surgical procedure can be performed even more quickly and safely.

Fig. 1 shows an example of a movement of the microscope head 2 from a first position (shown as microscope 1' and microscope head 2' in dashed lines) to a second position of the microscope head 2 and additional schematic vectors between the target surgical area and the optical system 4 as well as (focus) vectors along the movement of the microscope head 2.

The first focal point 8 in the first position of the microscope 1' is located on the optical axis 10. The length of the vector is specified by the control unit 20 as the focal length for the focus system 6. If the microscope head 2' is now moved to the second the control unit 22 continuously calculates the focus/ focal point 8 for the focus system 6 based on the kinematics of the surgical microscope 1, specifically of the microscope head 2 and the microscope arm 16. This way, the focus on the target surgical area is maintained even during complex movements and the surgeon. At all times, a selected target surgical area is maintained in focus.

The microscope 1 further comprises a data provision unit in the form of a memory unit 27 in which 3D recording data 3DA (not shown here) are stored. That way, it is possible for a surgeon, after registration of the 3D recording data 3DA with respect to the patient P, to set a target surgical area with a desired focal point in the digital 3D recording data 3DA (on the digital side, the position of the microscope head 2 and of the virtually defined target surgical area, which corresponds to the real target surgical area on the basis of the previous registration, is known), so that the control unit 22 calculates the virtual focus to be set, transfers it to the real world and controls the focus system 6 accordingly, in order to precisely set this focus or the previously calculated focal length. Preoperative three-dimensional imaging data 3DA, such as MRI images or CT images, can be used for the surgery using the microscope 1 and a surgery is further improved. The surgical target area may be defined based on preoperative 3D imaging data of the patient P, the preoperative 3D imaging data 3DA being registered to the patient P before a surgical procedure.

In Fig. 1, a further, different, exemplary focal point 8' is also defined, which does not lie on the optical (microscope) axis 10. This focal point 8' can also be set via the 3D recording data. In this case, the same focus calculations based on the kinematics of the microscope 1 as described above, apply.

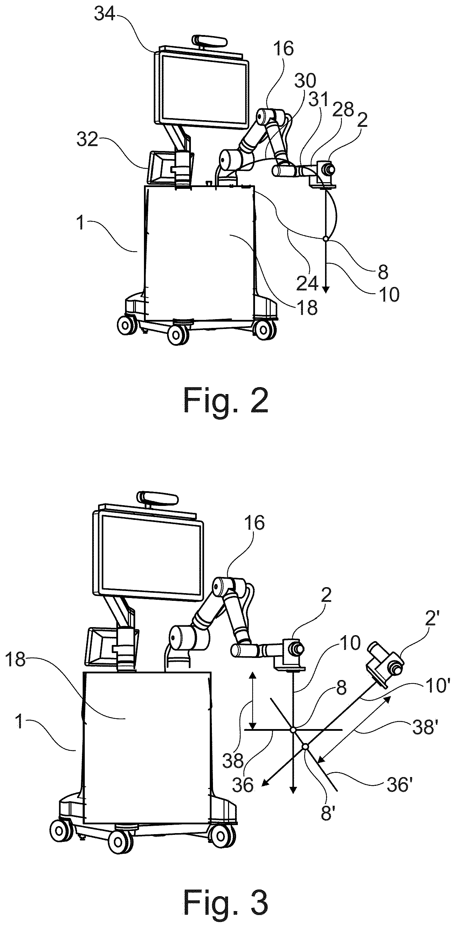

Fig. 2 shows a perspective view of the medical surgical microscope 1 of the preferred embodiment of the disclosure with an indicated transformation 24 of the position of the first focal point 8 of a local coordinate system of a microscope head 2 into a local coordinate system of a microscope base 18.

The microscope head 2 is connected to the microscope base 18 via the movable microscope arm 16. The vertical arrow in Fig. 2 corresponds to the optical axis 10 of the microscope head 2 which also crosses the focal point 8, in other words the focal point 8 is located on the optical axis 10. Fig. 2 shows the (virtual) transformation 24 of the position of the first focal point 8 of a local coordinate system of the microscope head 2 into a local coordinate system of the microscope base 18. This transformation 24 is calculated based on the focal length (distance from the focal point 8 and the microscope head 2, more specifically, the optical system 4 of the microscope head 2), a first transformation 28 from the local coordinate system of the microscope head 2 to a local coordinate system of a microscope flange 31 of the microscope arm 16, and calculate a second transformation 30 from the microscope flange 31 to the microscope base 18, by the control unit 22. Said transformations 24, 28, 30 are shown schematically in Fig. 2.

Furthermore, the surgical microscope 1 comprises an input device 32 for the surgeon/ operator as well as a display 34 which visually shows the information about the surgical process.

Fig. 3 shows a perspective view of the medical surgical microscope 1 in the preferred embodiment of the disclosure with a first and a second position of the microscope head 2, 2' as well as a respective first focal plane 36 and a second focal plane 36'. The setup of the surgical microscope 1 in Fig. 3 is the same as the surgical microscope 1 shown in Fig. 2. In addition, the microscope head 2 is shown in a first position and first orientation with a first vertical optical axis 10. The optical axis 10 is perpendicular to the first focal plane 36 in which the focal point 8 is located. The distance between the microscope head 2, specifically the optical system 4 of the microscope head 2, and the focal plane 36 is referenced as a focal length 38. A movement of the microscope head 2 is indicated by the microscope head 2' with a second position and a second orientation. Since the microscope head 2' has been rotated (with a different orientation) the new second optical axis 10' is not vertical. Therefore, the second focal plane 36' is also rotated relative to the first focal plane 36. In particular, the second focal plane 36' is rotated around the first focal point 8, in other words the first focal point 8 as well as the second focal point 8' are located on the second focal plane 36'. The control unit 22 computes he second focal point 8' by determining the second focal plane 36' that crosses the first focal point 8 and that is also perpendicular to the second optical axis 10' of the microscope head 2' in the second position and orientation based on the kinematics of the microscope head 2, 2' and/or the microscope arm 16. A new focal length 36' according to the second position and orientation of the microscope head 2' is also calculated by the control unit 22.

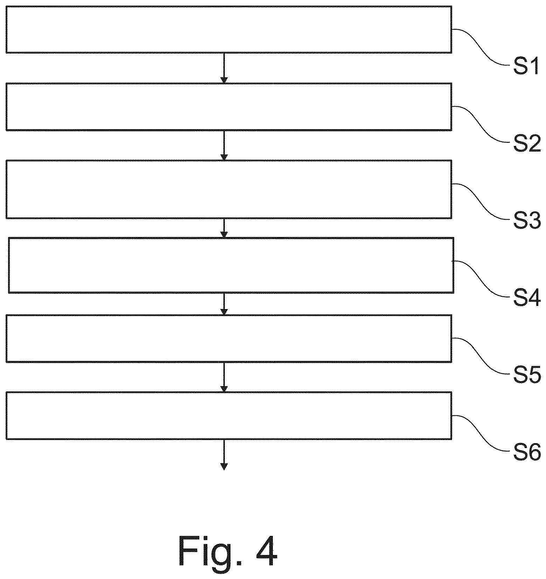

Fig. 4 shows in a flow chart a method for adjusting the focus on a target surgical area according to a preferred embodiment of the present disclosure for a medical surgical microscope 1 according to the present disclosure.

In a first step S1 the method is detecting a first focal point 8 of a focus system 6 of an optical system 4 in a first position and orientation of a movable microscope head 2 by a control unit 22.

In a following second step S2 the method is determining, by the control unit 22, of a first focal plane 36 that intersects the first focal point 8 and is perpendicular to an optical axis 10 of the microscope head 2 in the first position and/or orientation.

In step S3, the method is focusing, by a focusing system (6), on the first focal point 8.

In a following step S4, a moving, preferably by a movable robotic microscope arm 16, the microscope head 2 from the first position and/or orientation to a second position and/or orientation is conducted.

In step S5, Computing of a second focal point 8' by determining a second focal plane 36' that intersects the first focal point (8) and is perpendicular to an optical axis 10' of the microscope head 2' in the second position and/or orientation in which the second focal point 8' is located, is done.

Finally, in step S6, adjusting of the focus of the focus(-ing) system 6 to the second focal point 8' is performed.

### List of reference signs:

- 1, 1': surgical microscope
- 2, 2': microscope head
- 4: optical system
- 6: focus system
- 8, 8': focal point
- 10, 10': optical axis
- 16: microscope arm
- 18: microscope base
- 22: control unit
- 24: (main) transformation
- 27: memory unit
- 28: first transformation
- 30: second transformation
- 31: microscope flange
- 32: input device
- 34: display
- 36, 36': first, second focal plane
- 38, 38': first, second focal length

- S1: step detecting a first focal point
- S2: step determining a first focal plane
- S3: step focusing on the first focal point
- S4: step moving the microscope head
- S5: step computing a second focal point
- S6: step adjusting the focus

## Claims

1. A medical surgical microscope (1) for a surgical procedure on a patient
(P), comprising:
a moveable microscope head (2) with an optical system (4) with a focus system (6) for focus adjustment to a focal point (8), the microscope (1) being adapted to provide an optical magnification of a target surgical area, in particular in the direction of a first optical axis (10), and to generate a digital microscope image, preferably by means of a microscope sensing unit,
a movable robotic microscope arm (16) that is connected to a microscope base (18) to which the movable microscope head (2) is connected that is adapted to actively adjust a position and/or an orientation of the microscope head (2), and
a control unit (22) that is at least adapted to control the focus system (6) and the movable robotic microscope arm (16),
**characterized in that** the control unit (22) is further adapted to:
detect a first focal point (8) of the focus system (6) in a first position and orientation of the microscope head (2), wherein the first focal point (8) is located in a first focal plane (36) that is perpendicular to the optical axis (10) of the microscope head (2) in the first position and/or orientation,
move the microscope head (2) into a second position and/or orientation,
compute a second focal point (8') by determining a second focal plane (36') that intersects the first focal point (8) and is perpendicular to the optical axis (10') of the microscope head (2') in the second position and/or orientation in which the second focal point (8') is located, and
adjust the focus of the focusing system (6) to the second focal point (8').

2. The medical surgical microscope (1) according to claim 1, **characterized in that** the control unit (22) is further adapted to transform the position of the first focal point (8) of a local coordinate system of the microscope head (2) into a local coordinate system of the microscope base (18), in order to continuously calculate the position of the first focal point (8) relative to the microscope base (18).

3. The medical surgical microscope (1) according to claim 2, **characterized in that** the control unit (22) is adapted to calculate the transformation of the first focal point (8) of the local coordinate system of the microscope head (2) into the local coordinate system of the microscope base (18) by calculating a first transformation (28) from the local coordinate system of the microscope head (2) to a local coordinate system of a microscope flange (31), and calculate a second transformation (30) from the microscope flange (31) to the microscope base (18), in order to enhance a calculation process.

4. The medical surgical microscope (1) according to claim 1, **characterized in that** the control unit (22) is adapted to calculate a focal length (38; 38') by calculating a distance between the optical system (4) of the microscope head (2) and the focal plane (36; 36') in which the current focal point (8; 8') is located.

5. The medical surgical microscope (1) according to claim 4, **characterized in that** the focus system (6) is adapted to adjust the focus by explicitly setting the focus to the focal length (36') of the second focal point (8') calculated by the control unit (22).

6. The medical surgical microscope (1) according to any one of the preceding claims, **characterized in that** the control unit (22) is adapted to set, based on a calculated focal length (38') of the second position and/or orientation of the microscope head (2'), a minimum focal length and/or a maximum focal length as focus limiters, and the control unit (22) is adapted to perform a contrast focusing within the focus limiters.

7. The medical surgical microscope (1) according to claim 6, **characterized in that** a minimum focal length and a maximum focal length is set, wherein
the minimum focal length and the maximum focal length are equidistant to the focal length (38') calculated by the control unit (22), or
a difference between the maximum focal length and the focal length (38') is smaller than a difference between the focal length (38') and the minimum focal length, or
a difference between the maximum focal length and the focal length (38') is larger than a difference between the focal length (38') and the minimum focal length.

8. The medical surgical microscope (1) according to any one of the preceding claims, **characterized in that** the focus system (6) is adapted to adjust the focus based on a velocity information of the robotic microscope head (2) calculated by the control unit (22), preferably by using the first position and orientation of the microscope head (2) and by integrating an velocity vector as well as a rotation velocity.

9. The medical surgical microscope (1) according to any one of the preceding claims, **characterized in that** the control unit (22) is adapted to calculate the current focal length (38') of a moving microscope head (2') in real time and to continuously adjust the focal length (38') of the focus system (6) accordingly.

10. The medical surgical microscope (1) according to any one of the preceding claims, **characterized in that** the control unit (22) is adapted to control a focus motor of the focus system (6) via a velocity control or via a current control in order to adjust the focus of the focusing system (6).

11. Method for adjusting the focus on a target surgical area, in particular for a medical surgical microscope (1) according to any one of the preceding claims,
**characterized by** the steps:
- Detecting (S1) a first focal point (8) of a focus system (6) of an optical system (4) in a first position and orientation of a movable microscope head (2) by a control unit (22);
- Determining (S2), by the control unit (22), of a first focal plane (36) that intersects the first focal point (8) and is perpendicular to an optical axis (10) of the microscope head (2) in the first position and/or orientation;
- Focusing (S3), by a focusing system (6), on the first focal point (8);
- Moving (S4), preferably by a movable robotic microscope arm (16), the microscope head (2) from the first position and/or orientation to a second position and/or orientation;
- Computing (S5) a second focal point (8') by determining a second focal plane (36') that intersects the first focal point (8) and is perpendicular to an optical axis (10') of the microscope head (2') in the second position and/or orientation in which the second focal point (8') is located; and
- Adjusting (S6) the focus of the focusing system (6) to the second focal point (8').

12. Method for adjusting the focus according to claim 11, **characterized by** the additional steps of:
- Calculating a focal length (38; 38') by calculating a distance between the optical system (4) of the microscope head (2; 2') and the focal plane (36; 36') in which the first or second focal point (8; 8') is located
- Adjusting the focus by explicitly setting the focus to the focal length (38; 38') of the first or second focal point (8, 8').

13. A computer readable storage medium comprising instructions which, when executed by a computer, causes the computer to perform the method steps according to claim 11 or 12.

14. A computer program comprising instructions which, when executed by a computer, causes the computer to perform the method steps according to claim 11 or 12.
